# FASCICULE DE BREVET EUROPEEN

(11) **EP 0 679 392 B1**
(45) Date de publication et mention de la délivrance du brevet: **12.12.2001**
(21) Numéro de dépôt: 95400949.4
(22) Date de dépôt: 26.04.1995
(51) Int. Cl.: A61K 9/70, A61L 24/04

(54) **Nouvelle forme galénique pour l'administration transdermique**
Arzneiform zur transdermalen Verabreichung
Pharmaceutical form for transdermal administration

(30) Priorité: 29.04.1994 FR 9405272
(43) Date de publication de la demande: 02.11.1995
(73) Titulaire: LABORATOIRE L. LAFON, 94701 Maisons Alfort (FR)
(72) Inventeur: Laurent, Philippe, F-69600 Oulins (FR)
(74) Mandataire: Le Guen, Gérard

(56) Documents cités:
- EP-A- 0 164 999
- EP-A- 0 289 900
- EP-A- 0 409 550
- EP-A- 0 521 455
- EP-A- 0 560 014
- DE-A- 2 140 491
- US-A- 3 836 647

## Description

La présente invention concerne une nouvelle forme galénique pour l'administration transdermique d'un principe actif.

On a développé dans les années 1980 des systèmes transdermiques qui sont appliqués sur une surface délimitée de la peau et qui servent de support ou de véhicule à un ou plusieurs principes actifs, destinés généralement à exercer une action générale après libération et passage à travers la barrière cutanée.

Ces systèmes, appelés généralement "patchs transdermiques", présentent par rapport aux formes dermatologiques classiques telles que onguents, pommades, gels, solutions, lotions, un certain nombre d'avantages:
- passage direct et continu dans la circulation générale,
- suppression du premier passage hépatique et/ou des dégradations dans le tractus digestif avec pour conséquence une diminution des effets secondaires,
- prolongation de la durée d'action,
- maintien d'un taux constant de principes actifs dans le plasma,
- augmentation de l'observance par diminution de la fréquence des prises,
- diminution des variations inter-individuelles,
- maîtrise de la dose administrée grâce à un système matriciel ou membranaire à réservoir,
- obtention d'une concentration constante de principe actif durant la durée de l'application.

Malgré le degré d'innovation apporté par ces systèmes, il n'existe aujourd'hui que très peu de spécialités sous cette forme. Ceci est dû au fait que ces dispositifs exigent :
. une technologie de fabrication très sophistiquée,
. des sites de production rares qui appartiennent à quelques grands groupes qui en ont le monopole,
. ceci entraîne un coût élevé de fabrication et un prix de revient et de vente important. Ces systèmes sont en définitif réservés à des produits chers.

Par ailleurs, US 3 836 647 décrit une composition pour la protection de la peau ou son traitement, qui comprend un principe actif, une composition polymère adhésive à base de silicones et un solvant.

Quant à EP 0 560 014, il se rapporte à un film biodégradable protecteur de la peau comprenant un principe actif, une composition formée d'un polymère thermoplastique et un solvant organique.

La présente invention vise à fournir de nouvelles formes galéniques pour l'administration transdermique d'un principe actif
- qui soient très simples à mettre en oeuvre, ne demandent pas d'installations industrielles lourdes, compliquées et onéreuses,
- qui soient polyvalentes : à la fois sur le plan de la formulation que sur les modalités d'application lors de l'utilisation,
- qui soient intéressantes sur le plan économique avec un moindre coût de réalisation.

A cet effet, la présente invention a pour objet une composition destinée à former sur la peau une pellicule pour l'administration transdermique d'un principe actif, qui comprend :
a) un principe actif lipophile
b) de 5 à 60 % en poids, et avantageusement de 5 à 25 % en poids, d'une composition polymère adhésive à base de silicone
c) de 5 à 25 % en poids d'un promoteur d'absorption et
d) de 25 à 95 % en poids, et avantageusement de 50 à 95 % en poids, d'un solvant volatil.

La présente invention a également pour objet:
- l'utilisation d'une composition qui comprend :
   a) un principe actif lipophile
   b) de 5 à 60 % en poids, et avantageusement de 5 à 25 % en poids, d'une composition polymère adhésive à base de silicone
   c) de 5 à 25 % en poids d'un promoteur d'absorption et
   d) de 25 à 95 % en poids, et avantageusement de 50 à 95 % en poids, d'un solvant volatil
pour la fabrication d'un médicament pour l'administration transdermique du principe actif.

Dans la présente invention par principe actif on désigne principalement un médicament ou une substance ayant des propriétés thérapeutiques.

Ces médicaments sont notamment des vitamines lipophiles telles que les vitamines D et E et leurs dérivés, des hormones telles que la calcitonine, des stéroïdes tels que l'estradiol et ses esters et la prednisone, ou la nicotine.

Les pourcentages des principes actifs dans les compositions de l'invention dépendent évidemment de la nature du principe actif. Généralement les pourcentages sont de 0,01 à 10% en poids.

Selon l'invention, on entend par composition polymère à base de silicone une composition contenant aussi bien des polymères à base de silicones que des copolymères à base de silicones.

Ces silicones, qui seront désignés selon la nomenclature du dictionnaire de la CTFA (Cosmetic, Toiletry and Fragrance Association), comprennent notamment des huiles polydiméthylsiloxanes ou des huiles polydiméthylsiloxanes modifiées par des groupes organiques ioniques ou non ioniques.

Comme exemple d'huiles polydiméthylsiloxanes, on citera les diméthicones de formule : 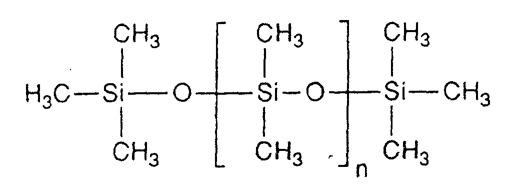 où n est un entier inférieur à 5000, et les diméthiconols qui sont des diméthylsilicones terminés par des groupes hydroxy.

Comme exemple de polydiméthylsiloxanes modifiés, on citera les diméthicone copolyols qui sont des polymères de diméthylsiloxane comportant des chaînes latérales polyoxyéthylène et/ou polyoxypropylène.

La composition polymère adhésive à base de silicone représente de préférence 9 à 12 % du poids de la composition.

Les promoteurs d'absorption peuvent être choisis notamment parmi le propylèneglycol, I'hexylèneglycol, le dipélargonate de propylène glycol, l'éther monoéthylique de glycéryle, le diéthylène glycol, des monoglycérides, du monooléate de glycérides éthoxylés (avec 8 à 10 motifs oxyde d'éthylène), l'azone (1-dodécyl azacycloheptane-2-one), le 2-(n-nonyl)-1,3-dioxolanne, le myristate d'isopropyle, le myristate d'octyle, le myristate de dodécyle, l'alcool myristylique, l'alcool laurylique, l'acide laurique, le lactate de lauryle, le terpinol, le 1-menthol, le d-limonène, la β-cyclodextrine et ses dérivés ou des agents tensioactifs tels que polysorbates, esters de sorbitan, esters de saccharose, acides gras, sels biliaires, ou encore des produits lipophiles et/ou hydrophiles et/ou amphiphiles tels que esters du polyglycérol, N-méthylpyrrolidone, glycérides polyglycosylés et lactate de cétyle.

Comme solvant volatil on peut utiliser notamment des polydiméthylcyclosiloxanes, c'est-à-dire des composés de formule : 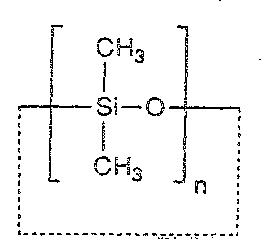 où n est en moyenne entre 3 et 6 et notamment des composés où n = 4 ou 5, ainsi que les polysiloxanes linéaires tels que l'hexaméthyl disiloxane ou des diméthicones de faible masse moléculaire.

On peut également utiliser d'autres solvants tels que éthanol, isopropanol, chloroforme, heptane, acétate d'éthyle.

Le solvant représente de préférence de 65 à 85 % du poids de la composition.

La composition selon l'invention peut être contenue dans un appareil distributeur qui délivre des doses définies et reproductibles de composition. Par exemple l'appareil distributeur délivre une goutte de composition et cette goutte peut être étalée sur la peau à l'aide d'un pinceau ou d'une bille que l'on fait rouler sur la peau.

La présente invention trouve une utilisation particulièrement intéressante pour l'administration transdermique de vitamine D₃ (cholecalciférol) et de ses dérivés hydroxylés .

Des études récentes tendent à montrer que toutes les populations des pays occidentaux et en particulier les pays européens sont carencées en hiver en vitamine D. Le phénomène est de moindre importance aux Etats-Unis et dans les Pays Scandinaves qui ont une alimentation enrichie en vitamine D₃.

En général, l'hypovitaminose a été observée chez les personnes âgées de tous les pays et se manifeste par une ostéomalacie et des phénomènes anormaux dans la chimie des os.

Les causes de carence sont :
. apport alimentaire insuffisant quantitativement et qualitativement : oeufs, beurre, foie, poissons gras.
. le manque d'ensoleillement car la synthèse cutanée se fait sous l'effet des rayons U.V.. Cette source d'approvisionnement en vitamine D naturelle est fortement dépendante des conditions climatiques.
. le syndrome de mal absorption : chez le sujet âgé il existe une diminution de l'absorption intestinale de la vitamine D du fait de la diminution des fonctions hépatique et rénale.

Actuellement les spécialités disponibles sur le marché se présentent essentiellement sous des formes galéniques pour voie orale et quelques unes pour la voie injectable (IM). Or, la voie orale n'est pas toujours bien assimilée et la voie injectable pas toujours acceptée par les personnes âgées.

La présente invention a donc plus spécifiquement pour objet une composition destinée à former sur la peau une pellicule pour l'administration transdermique de vitamine D₃ ou un dérivé hydroxylé de vitamine D₃ et qui comprend :
a) de la vitamine D₃ ou un dérivé hydroxylé de vitamine D₃
b) de 5 à 60 % en poids et, de préférence, de 9 à 12 % en poids d'une composition polymère adhésive à base de silicones
c) de 5 à 25 % en poids d'un promoteur d'absorption et
d) de 25 à 95 % en poids et, de préférence, de 65 à 85 % en poids d'un solvant volatil.

On donnera ci-après des exemples de compositions selon l'invention.

### Exemples 1 à 11 : Compositions à base de vitamine D₃.

On a préparé les compositions figurant dans le tableau ci-après en mélangeant les différents constituants jusqu'à obtenir un mélange homogène.

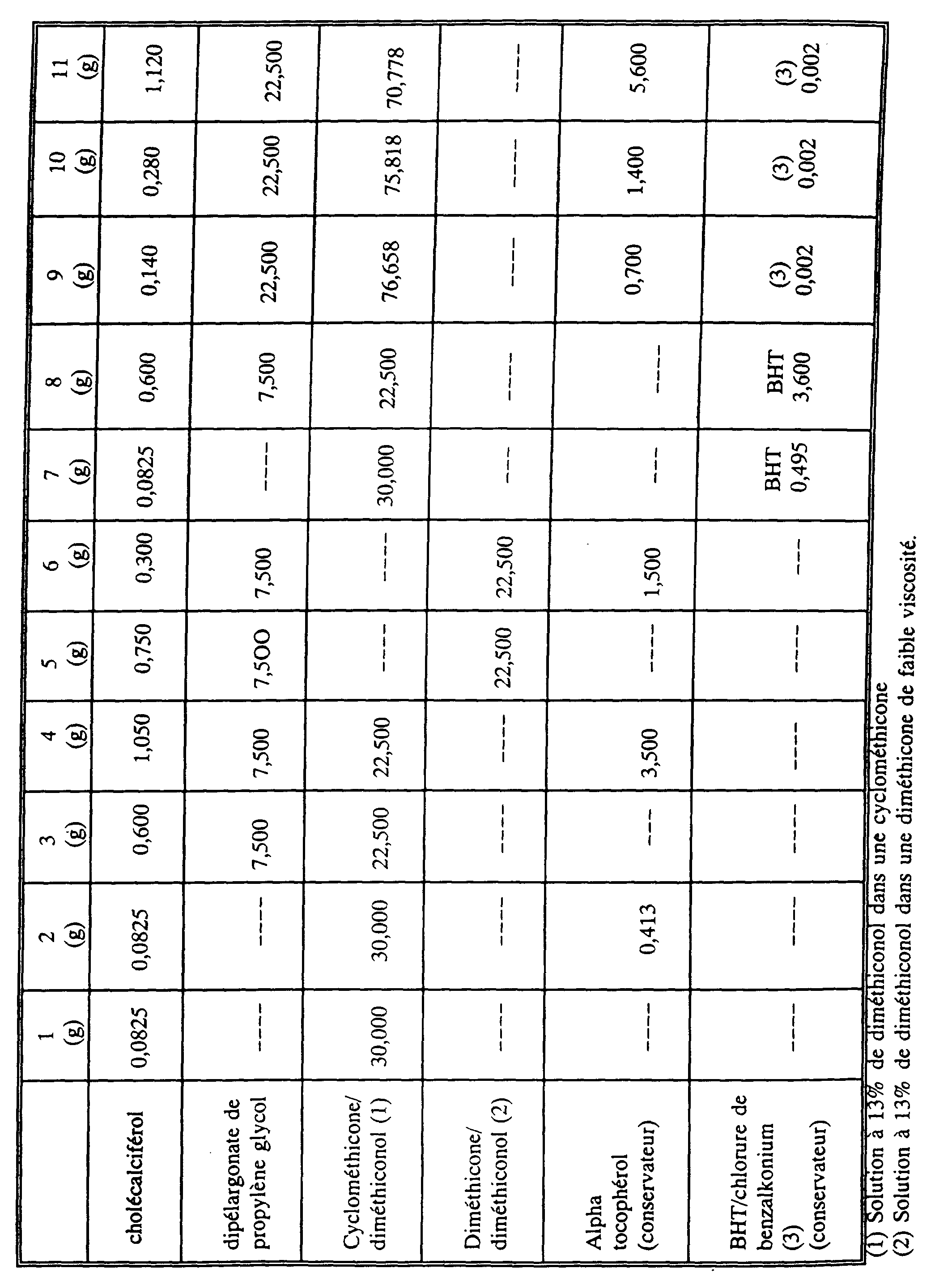

Au moment de l'utilisation, à l'aide d'un système applicateur, on dépose une goutte dans une composition sur la peau et on étale sur une surface déterminée.

La pellicule transdermique se forme après évaporation du solvant silicone.

### Exemple 12 : Composition à base de 1,2,5-dihydroxycholécalciférol.

| | | |
|---|---|---|
| A. | 1,2,5-dihydroxycholécalciférol | 2 µg |
| B. | Monoéthyléther de diéthylène glycol | 2,50 % |
| C. | Monooléate de glycéryle | 1,25% |
| D. | Dipélargonate de propylène glycol | 1,25% |
| E. | Diméthylpolysiloxane | 55,00% |
| F. | Cyclométhicone | QSP 100 µl |

### Exemple 13 : Composition à base de calcitonine.

| | | |
|---|---|---|
| A. | Calcitonine | 100 UI |
| B. | Azone | 10 % |
| C. | Copolymère de polyacryl amide d'isoparaffine et d'alcools lauryliques polyoxyéthylénés | 5 % |
| D. | Propylèneglycol | 20 % |
| E. | Diméthicone et diméthiconol | 20 % |
| F. | Polydiméthylcyclosiloxane | QSP 50 microlitres |

### Exemple 14 : Composition à base d'ester d'estradiol.

| | | |
|---|---|---|
| A. | Ester propionique et nicotinique d'estradiol | 1,3 mg |
| B. | Monoéthyléther de diéthylène glycol | 5 % |
| C. | Monooléate de glycéryle | 2,5 % |
| D. | Dipélargonate de propylène glycol | 2,5 % |
| E. | Diméthicone et diméthiconol | 55 % |
| F. | Polydiméthylcyclosiloxane | QSP 100 µl |

### Exemple 15 : Composition à base de prednisone.

| | | |
|---|---|---|
| A. | Prednisone | 2 mg |
| B. | Azone | 5 % |
| C. | Bétacyclodextrine | 10 % |
| D. | Cyclométhicone et diméthiconol | 20 % |
| E. | Ethanol | 10 % |
| F. | Polydiméthylcyclosiloxane | QSP 100 µl |

### Exemple 16 : Composition à base de calcitonine

| | | |
|---|---|---|
| A. | Calcitonine | 100 UI |
| B. | Azone | 10 % |
| C. | Copolymère de polyacrylamide d'isoparaffine et d'alcools lauryliques polyoxyéthylénés | 5 % |
| D. | Propylène glycol | 5 % |
| E. | Cyclométhicone et diméthiconol | 40 % |
| F. | Ethanol | 10% |
| G. | Polydiméthylcyclosiloxane | QSP 100 µl |

### Exemples 17 à 22

On a préparé les compositions suivantes pour l'administration transdermique de 17 β-estradiol.

| **Exemples** | **17** | **18** | **19** | **20** | **21** | **22** |
|---|---|---|---|---|---|---|
| 17 β-estradiol | 0,250 g | 0,250 g | 0,250 g | 0,250 g | 0,250 g | 0,250 g |
| DPPG ⁽¹⁾ | 10,00 g | 10,00 g | 10,00 g | 20,00 g | 20,00 g | 20,00 g |
| SEPA ⁽²⁾ | | 2,00 g | 5,00 g | | 2,00 g | 5,00 g |
| Ethanol | 20,00 g | 20,00 g | 20,00 g | 20,00 g | 20,00 g | 20,00 g |
| Silicone 1401 ⁽³⁾ QSP | 100,00g | 100,00g | 100,00g | 100,00g | 100,00g | 100,00g |

| | | | | | | |
|---|---|---|---|---|---|---|
| ⁽¹⁾ Dipélargonate de propylèneglycol | | | | | | |
| ⁽²⁾ 2-(n-nonyl)-1,3-dioxolanne | | | | | | |
| ⁽³⁾ Solution à 13 % de diméthiconol dans une cyclométhicone. | | | | | | |

On a effectué avec ces compositions une étude de la diffusion à travers la peau humaine in vitro.

La méthode utilisée est la suivante.

Une quantité exacte de composition mesurée volumétriquement (10 µl) est appliquée sur une biopsie de peau humaine dermatomée (épaisseur constante de 350 µm) placée dans une cellule de diffusion de type statique dite de Franz®. Le contact est maintenu durant 2, 4, 6, 8, 10 et 24 heures. Les échantillons de peau humaine proviennent de pièces anatomiques prélevées au niveau abdominal et/ou mammaire en cours d'intervention de chirurgie plastique.

Le liquide de survie est un tampon phosphate de pH 7,4 renfermant de l'albumine (sérum albumine bovine 15 g/l). Au terme de chaque temps de contact, le liquide du compartiment dermique est prélevé et le principe actif qu'il contient est dosé.

Au terme des 24 heures de contact la surface cutanée est lavée. Le principe actif persistant à la surface de la peau et entraîné dans les liquides de lavage est quantifié.

Les résultats obtenus au bout de 24 heures sont donnés dans le tableau suivant en % absorbés de la dose appliquée.

| **Exemples** | |
|---|---|
| 17 | 2,1 ± 1,0 |
| 18 | 2,7 ± 1,1 |
| 19 | 3,8 ± 0,9 |
| 20 | 4,4 ± 1,7 |
| 21 | 4,5 ± 2,5 |
| 22 | 9,4 ± 3,1 |

### Exemples 23 à 26

On a préparé les compositions suivantes pour l'administration transdermique de cholecalciférol.

| **Exemples** | **23** | **24** | **25** | **26** |
|---|---|---|---|---|
| Cholécalciférol | 0,534 g | 0,534 g | 0,534 g | 0,534 g |
| Alpha tocophérol | 2,800 g | 2,800 g | 2,800 g | 2,800 g |
| DPPG ⁽¹⁾ | 22,500 g | 22,500 g | 22,500 g | 22,500 g |
| SEPA™ ⁽²⁾ | 0,000 g | 2,000 g | 5,000 g | 10,000 g |
| Parahydroxybenzoate de méthyle | 0,250 g | | | |
| Parahydroxybenzoate de propyle | 0,100 g | | | |
| Ethanol | 0,650 g | | | |
| Silicone ⁽³⁾ QSP | 100,000 g | 100,000 g | 100,000 g | 100,000 g |

| | | | | |
|---|---|---|---|---|
| ¹⁾ Dipélargonate de propylèneglycol | | | | |
| ⁽²⁾ 2-(n-nonyl)-1,3-dioxolanne | | | | |
| ⁽³⁾ Solution à 13 % de diméthiconol dans une cyclométhicone. | | | | |

On a opéré comme avec les compositions des exemples 17 à 22 en déposant 10 mg de composition (53,40 µg de cholécalciférol).

Les résultats sont donnés dans le tableau ci-après :

| **Quantités en µg de vitamine D3 absorbées (± Sd)** | | **2 Heures** | **4 Heures** | **6 heures** | **8 Heures** | **10 Heures** | **24 Heures** |
|---|---|---|---|---|---|---|---|
| Exemple 23 : | (µg) | 1,0820 | 1,6223 | 2,1175 | 2,5170 | 2,8520 | 4,4525 |
| | (±) | 0,3667 | 0,4696 | 0,6116 | 0,7228 | 0,8417 | 1,1364 |
| Exemple 24 : | (µg) | 1,1173 | 1,52220 | 1,8880 | 2,1758 | 2,4260 | 3,6465 |
| | (±) | 0,2789 | 0,3773 | 0,4594 | 0,5138 | 0,5549 | 0,6630 |
| Exemple 25 : | (µg) | 1,3078 | 1,8285 | 2,3330 | 2,7273 | 3,0893 | 4,8973 |
| | (±) | 0,5660 | 0,7634 | 0,9587 | 1,1191 | 1,2645 | 1,8922 |
| Exemple 26 : | (µg) | 1,1983 | 1,8933 | 2,4513 | 2,8553 | 3,2080 | 4,7830 |
| | (±) | 0,5044 | 0,4308 | 0,4390 | 0,4196 | 0,3928 | 0,3038 |

## Revendications

1. Composition destinée à former sur la peau une pellicule pour l'administration transdermique d'un principe actif, qui comprend :
a) un principe actif lipophile
b) de 5 à 60 % en poids d'une composition polymère adhésive à base de silicone
c) de 5 à 25 % en poids d'un promoteur d'absorption et
d) de 25 à 95 % en poids d'un solvant volatil.

2. Composition selon la revendication 1, dans laquelle le principe actif lipophile est choisi parmi les vitamines lipophiles, les hormones et les stéroïdes.

3. Composition selon la revendication 1 ou 2, dans laquelle le principe actif est choisi parmi la vitamine D₃ et ses dérivés hydroxylés.

4. Composition selon les revendications 1 à 3, dans laquelle la composition polymère adhésive comprend des polysiloxanes.

5. Composition selon la revendicaiton 4, dans laquelle le solvant est un polydiméthylcyclosiloxane.

6. Composition selon la revendication 3 destinée à former sur la peau une pellicule pour l'administration transdermique de vitamine D₃ ou un dérivé hydroxylé de vitamine D₃ et qui comprend :
a) de la vitamine D₃ ou un dérivé hydroxylé de vitamine D₃
b) de 9 à 12 % en poids d'une composition polymère adhésive à base de silicone
c) de 5 à 25 % en poids d'un promoteur d'absorption et
d) de 65 à 85 % en poids d'un solvant volatil.

7. Utilisation d'une composition qui comprend :
a) un principe actif lipophile
b) de 5 à 60 % en poids d'une composition polymère adhésive à base de silicone
c) de 5 à 25 % en poids d'un promoteur d'absorption et
d) de 25 à 95 % en poids d'un solvant volatil
pour la fabrication d'un médicament pour l'administration transdermique du principe actif.

## Patentansprüche

1. Zusammensetzung, die dazu bestimmt ist, auf der Haut einen Film zur transdermalen Verabreichung eines Wirkstoffs zu bilden, umfassend:
a) einen lipophilen Wirkstoff
b) 5 bis 60 Gew.-% einer haftenden polymeren Zusammensetzung auf Siliconbasis
c) 5 bis 25 Gew.-% eines Absorptionspromotors und
d) 25 bis 95 Gew.-% eines flüchtigen Lösungsmittels.

2. Zusammensetzung nach Anspruch 1, in der der lipophile Wirkstoff aus den lipophilen Vitaminen, den Hormonen und den Steroiden gewählt ist.

3. Zusammensetzung nach Anspruch 1 oder 2, in der der lipophile Wirkstoff aus dem Vitamin D₃ und seinen hydroxylierten Derivaten gewählt ist.

4. Zusammensetzung nach Anspruch 1 bis 3, in der die haftende polymere Zusammensetzung Polysiloxane enthält.

5. Zusammensetzung nach Anspruch 4, in der das Lösungsmittel ein Polydimethylcyclosiloxan ist.

6. Zusammensetzung nach Anspruch 3, die dazu bestimmt ist, auf der Haut einen Film zur transdermalen Verabreichung von Vitamin D₃ oder eines hydroxylierten Derivats des Vitamins D₃ zu bilden, umfassend:
a) Vitamin D₃ oder ein hydroxyliertes Derivat des Vitamins D₃
b) 9 bis 12 Gew.-% einer haftenden polymeren Zusammensetzung auf Siliconbasis
c) 5 bis 25 Gew.-% eines Absorptionspromotors und
d) 65 bis 85 Gew.-% eines flüchtigen Lösungsmittels.

7. Anwendung einer Zusammensetzung, die enthält:
a) einen lipophilen Wirkstoff
b) 5 bis 60 Gew.-% einer haftenden polymeren Zusammensetzung auf Siliconbasis
c) 5 bis 25 Gew.-% eines Absorptionspromotors und
d) 25 bis 95 Gew.-% eines flüchtigen Lösungsmittels
zur Herstellung eines Medikaments zur transdermalen Verabreichung des Wirkstoffs.

## Claims

1. Composition intended to form a film on the skin for the transdermal administration of an active ingredient, comprising:
a) a lipophilic active ingredient,
b) from 5 to 60% by weight of a silicone-based adhesive polymer composition,
c) from 5 to 25% by weight of an absorption promoter, and
d) from 25 to 95% by weight of a volatile solvent.

2. Composition as claimed in Claim 1, in which the lipophilic active ingredient is chosen from amongst lipophilic vitamins, hormones and steroids.

3. Composition as claimed in Claim 1 or 2, in which the active ingredient is chosen from amongst vitamin D₃ and its hydroxyl derivatives.

4. Composition as claimed in Claims 1 to 3, in which the adhesive polymer composition comprises polysiloxanes.

5. Composition as claimed in Claim 4, in which the solvent is a polydimethylcyclosiloxane.

6. Composition as claimed in Claim 3 intended to form a film on the skin for the transdermal administration of vitamin D₃ or a hydroxyl derivative of vitamin D₃ and comprising:
a) vitamin D₃ or a hydroxyl derivative of vitamin D₃,
b) from 9 to 12% by weight of a silicone-based adhesive polymer composition,
c) from 5 to 25% by weight of an absorption promoter, and
d) from 65 to 85% by weight of a volatile solvent.

7. Use of a composition comprising:
a) a lipophilic active ingredient,
b) from 5 to 60% by weight of a silicone-based adhesive polymer composition,
c) from 5 to 25% by weight of an absorption promoter, and
d) from 25 to 95% by weight of a volatile solvent
for the production of a medicament for the transdermal administration of the active ingredient.
